# FASCICULE DE BREVET EUROPEEN

(11) **EP 1 273 565 B1**
(45) Date de publication et mention de la délivrance du brevet: **23.11.2005**
(21) Numéro de dépôt: 02291695.1
(22) Date de dépôt: 05.07.2002
(51) Int. Cl.: C07C 51/56, C07C 57/04

(54) **Procédé de préparation d'anhydride (meth)acrylique**
Verfahren zur Herstellung von (Meth)Acrylsäureanhydrid
Process for the preparation of (meth)acrylic anhydride

(30) Priorité: 06.07.2001 FR 0109009
(43) Date de publication de la demande: 08.01.2003
(73) Titulaire: Arkema, 92800 Puteaux (FR)
(72) Inventeur: Dupont, Bernard, 57150 Creutzwald (FR); Paul, Jean-Michel, 57070 Metz (FR)
(74) Mandataire: Hirsch & Associés

(56) Documents cités:
- EP-A- 0 004 641
- EP-A- 0 196 520
- US-A- 4 857 239

## Description

La présente invention concerne un procédé de préparation d'anhydride (méth)acrylique en discontinu.

Les anhydrides acrylique et méthacrylique sont des réactifs de choix dans la synthèse de thioesters (méth)acryliques, d'amides (méth)acryliques, d'esters (méth)acryliques, en particuliers d'alcools tertiaires qui ne peuvent être obtenus par les procédés classiques d'estérification/transestérification.

Ils sont aussi utilisés dans des réactions de polymérisation ou comme agents de réticulation.

On sait depuis longtemps qu'il est possible de préparer un anhydride par réaction d'anhydride acétique avec l'acide correspondant à l'anhydride désiré.

En 1934 déjà, le brevet français n° 784 458 décrivait la préparation des anhydrides propanoïque, butyrique et caproïque par réaction d'anhydride acétique avec l'acide propanoïque, l'acide butyrique et l'acide caproïque, respectivement.

En 1979, la demande de brevet européen n° 4 641 mettait à la disposition du public un procédé continu ou discontinu pour la préparation d'anhydrides d'acides carboxyliques tels que les anhydrides benzoïque, hexahydrobenzoïque et triméthylacétique, par réaction dans un appareillage de réaction/distillation, des acides correspondants avec l'anhydride acétique, de préférence dans des proportions stoechiométriques.

En 1986, la demande de brevet allemand n° 3 510 035 divulguait un procédé pour la préparation en continu d'anhydrides d'acides carboxyliques tels que l'anhydride acrylique ou méthacrylique par réaction, dans une colonne de distillation et en présence d'un catalyseur tel que l'acide sulfurique ou des acides sulfoniques ou phoshoriques, de l'anhydride acétique avec l'acide correspondant à l'anhydride désiré.

En 1987, la demande de brevet français n° 2 592 040 proposait un procédé pour la synthèse en discontinu d'anhydride (méth)acrylique par réaction d'anhydrique acétique avec de l'acide (méth)acrylique en présence d'inhibiteurs de polymérisation. Selon ce procédé, on fait d'abord réagir l'anhydride acétique et l'acide (méth)acrylique, on soutire l'acide acétique formé pendant la réaction, puis on effectue une distillation. Le rapport molaire entre l'acide (méth)acrylique et l'anhydride acétique est choisi entre 0,5 et 5, et de préférence entre 2 et 2,2.

La mise en oeuvre de ce procédé se heurte cependant à des problèmes de polymérisation. En outre, la quantité d'anhydride produit est limitée par la taille du réacteur et donc par la quantité de réactifs chargée dans ce réacteur.

EP-A-1231201, qui est un document sous l'article 54(3)(4)CBE, décrit un procédé de préparation d'anhydride d'acide carboxylique insaturé, comprenant la réaction d'un acide carboxylique insaturé et d'un d'anhydride d'acide acide carboxylique aliphatique inférieur en présence d'un catalyseur et d'un stabiliseur. L'exemple unique de ce document comprend la réaction d'acide méthacrylique et de la moitié de l'anhydride acétique utilisé dans un ballon. La réaction est commencée et le distillat contenant l'acide acétique est soutiré et la seconde partie de l'anhydride acétique est ajoutée goutte à goutte. Les ratios molaires initiaux de l'acide (méth)acrylique à l'anhydride acétique et de l'acide (méth)acrylique à l'anhydride acétique sont de 3.32 et de 1.66, respectivement.

La présente invention a donc pour but de proposer un procédé de préparation d'anhydride (méth)acrylique qui offre une productivité plus élevée et une diminution, voire une suppression, des risques de polymérisation.

L'invention a donc généralement pour objet un procédé de préparation d'anhydride (méth)acrylique en discontinu, dans lequel on fait réagir de l'anhydride acétique avec de l'acide (méth)acrylique et on élimine au moins en partie l'acide acétique au fur et à mesure de sa formation, et caractérisé en ce que l'acide acétique éliminé est remplacé au moins partiellement par introduction dans le milieu réactionnel, au cours de la réaction, d'anhydride acétique et/ou d'acide (méth)acrylique.

Ainsi, selon un premier mode de réalisation, l'invention fournit un procédé de préparation d'anhydride (méth)acrylique en discontinu, dans lequel on fait réagir de l'anhydride acétique avec de l'acide (méth)acrylique et on élimine au moins en partie l'acide acétique au fur et à mesure de sa formation, caractérisé en ce que l'acide acétique éliminé est remplacé au moins partiellement par introduction dans le milieu réactionnel, au cours de la réaction, d'anhydride acétique et/ou d'acide (méth)acrylique;
à l'exclusion du procédé dans lequel le rapport molaire global de l'acide (méth)acrylique à l'anhydride acétique est compris entre 1 et 2 et dans lequel le rapport molaire initial R₀ de l'acide (méth)acryligue à l'anhydride acétique est le double du rapport molaire global.

Ainsi, selon un second mode de réalisation, l'invention fournit un procédé de préparation d'anhydride (méth)acrylique en discontinu, dans lequel on fait réagir de l'anhydride acétique avec de l'acide (méth)acrylique et on élimine au moins en partie l'acide acétique au fur et à mesure de sa formation, caractérisé en ce que l'acide acétique éliminé est remplacé au moins partiellement par introduction dans le milieu réactionnel, au cours de la réaction, d'anhydride acétique et/ou d'acide (méth)acrylique;
à l'exclusion du procédé mettant en oeuvre un catalyseur et un inhibiteur.

Un tel procédé selon le premier ou second mode de réalisation permet une augmentation de la productivité supérieure à 35% par rapport aux procédés de l'art antérieur.

D'autres caractéristiques et avantages de l'invention vont maintenant être décrits en détail dans l'exposé qui suit.

### EXPOSÉ DÉTAILLÉ DE L'INVENTION

L'invention est basée sur la découverte suprenante qu'a faite la Demanderesse, à savoir, que grâce à une répartition judicieuse des réactifs, il est possible d'obtenir des gains importants de production par batch (en discontinu) d'anhydride (méth)acrylique et ceci, sans augmentation de la durée de la réaction.

Cette très forte augmentation de la production par batch est obtenue avec une masse initiale de réactifs (anhydride acétique et acide (méth)acrylique) identique.

Cette charge initiale est de préférence la charge maximale permise par le volume du réacteur.

Ainsi, selon l'invention, l'acide acétique, qui se forme par réaction de l'anhydride acétique et qui est éliminé au moins en partie au fur et à mesure de sa formation, est remplacé par l'un et/ou l'autre des réactifs.

En d'autres termes, on rajoute une quantité du ou des réactif (s) pour occuper la place libérée par l'élimination de l'acide acétique.

De préférence, on élimine tout l'acide acétique qui se forme pendant la réaction, au fur et à mesure de sa formation et par distillation.

Afin d'optimiser la production d'anhydride (méth)acrylique, il est souhaitable de remplacer, au moyen du ou des réactif(s), tout l'acide acétique éliminé.

En outre, une addition continue du ou des réactif(s) pendant toute la durée de la réaction est une variante préférable à une addition irrégulière.

Il est également préférable que cette addition suive, du plus près possible, l'élimination de l'acide acétique.

Ceci se traduit alors par une occupation pratiquement totale du volume du réacteur tout au long de la réaction.

De préférence, on n'ajoute qu'un seul des réactifs.

Le réactif ajouté est avantageusement l'anhydride acétique.

De plus, la charge initiale introduite dans le réacteur présente de préférence un rapport molaire initial R₀ de l'acide (méth)acrylique à l'anhydride acétique compris entre 2,5 et 11 et, en particulier, entre 9 et 11.

Le rapport molaire global R_{g} de l'acide (méth)acrylique à l'anhydride acétique est compris de préférence entre 0,5 et 5 et, en particulier, entre 1,8 et 2,2.

La réaction peut être mise en oeuvre dans un réacteur surmonté d'une colonne à distiller.

En général, le réacteur est agité et chauffé par circulation de fluide caloporteur dans une double enveloppe ou par recirculation au travers d'un échangeur de chaleur extérieur.

La colonne à distiller a de préférence une efficacité de séparation supérieure à 10 plateaux théoriques et en particulier supérieure à 12 plateaux théoriques. Ceci permet de minimiser les pertes en anhydride acétique via la première fraction de distillation, qui est alors constituée à plus de 99% par de l'acide acétique, d'opérer à faible taux de reflux (R/C inférieur ou égal à 2/1) et par conséquent de réduire le temps de la réaction et les risques de polymérisation qui augmentent avec le durée de la réaction.

Le garnissage de la colonne peut être un garnissage classique, en vrac ou structuré, ou un mélange de ces deux types de garnissage.

La température de réaction est généralement comprise entre 50 et 120°C, préférentiellement entre 85 et 105°C.

La pression est ajustée en fonction de la température de réaction choisie. En général, elle est comprise entre 20 et 200 mm Hg (0,0267 et 0,2666 bar).

La réaction peut être effectuée en mode dit « isobar », c'est-à-dire en fixant la pression et en laissant évoluer la température jusqu'à une valeur limite fixée de préférence entre 90 et 105°C, ou en mode « isotherme », à savoir, en fixant la température et en ajustant la pression dans l'installation tout au long de la réaction pour maintenir cette température.

La température en tête de colonne est avantageusement ajustée durant la réaction, en fonction de la pression, de façon à correspondre à la température de distillation de l'acide acétique.

En opérant ainsi, on obtient une fraction de tête contenant plus de 99% d'acide acétique.

Selon un mode de réalisation préféré de l'invention, la réaction entre l'anhydride acétique et l'acide (méth)acrylique est mise en oeuvre en présence d'au moins un inhibiteur de polymérisation.

En outre, on effectue de préférence une double stabilisation, en introduisant au moins un inhibiteur dans le réacteur et au moins un inhibiteur dans la colonne à distiller.

Les inhibiteurs doivent être actifs vis-à-vis de la polymérisation tout en étant inertes à l'égard des anhydrides et de l'acide (méth)acrylique.

Ainsi, on évite tout risque de polymérisation dans le réacteur et la colonne.

L'inhibiteur pour le réacteur est avantageusement choisi dans le groupe constitué par le 2,4-diméthyl 6-terbutyl phénol (« TOPANOL A »), le 2,6-diterbutyl paracrésol (« BHT ») et leurs mélanges.

L'inhibiteur de la colonne à distiller est avantageusement choisi dans le groupe constitué par l'hydroquinone (« HQ »), le le 2,4-diméthyl 6-terbutyl phénol, le 2,6-diterbutyl paracrésol, la phénotiazine et leurs mélanges.

Pour ce qui est des quantités à mettre en oeuvre, l'inhibiteur du réacteur est de préférence introduit dans la charge initiale des réactifs, à raison d'au moins 0,001 % (1000 ppm) en poids de la charge.

L'inhibiteur de la colonne à distiller est de préférence introduit dans la colonne à distiller tout au long de la réaction, par exemple en solution à 5% (en poids par rapport au poids total de la solution) dans de l'acide acétique. Le débit d'introduction de l'inhibiteur de la colonne est ajusté de façon à avoir moins de 1000 ppm d'inhibiteur dans le produit final du réacteur.

Un bullage d'air appauvri (8% d'oxygène et 92% d'azote en volumes) peut être effectué durant toute la réaction.

Le brut obtenu est en général parfaitement limpide, exempt de polymères et apte à être étêté par distillation sous pression réduite (par exemple de 20 mm Hg) afin de le débarrasser de l'excès d'acide acétique, d'acide (méth)acrylique et du composé mixte formé par réaction d'une mole d'acide (méth)acrylique avec une mole d'anhydride acétique.

Le procédé selon l'invention peut comprendre une étape ultérieure de distillation du produit brut obtenu, le cas échéant après étêtement, sur une colonne à distiller ou à l'aide d'un appareil à court temps de séjour tel qu'un évaporateur à film.

### Exemples

Les exemples suivants illustrent la présente invention sans toutefois en limiter la portée. Les pourcentages y sont exprimés en masses.

Les abbréviations suivantes y sont utilisées :
AMA : acide méthacrylique
AA : acide acrylique
AMA₂O : anhydride méthacrylique
AA₂O : anhydride acrylique
Ac₂O : anhydride acétique
AcOH : acide acétique
Mixte : H₂C=CHCOOOCCH₃, ou selon le cas, H₂C=C (CH₃)COOOCCH₃

### Exemple 1 (comparatif)

Dans un réacteur agité mécaniquement, chauffé par circulation d'huile thermostatée dans une double enveloppe, surmonté d'une colonne à distiller ayant un garnissage structuré Multiknit® ayant un efficacité de séparation de 12 plateaux théoriques, et pouvant fonctionner sous vide, on introduit 361 g (3,54 moles) d'Ac₂O et 639 g (7,43 moles) d'AMA (le rapport molaire R₀ est donc de 2,1).

On ajoute comme inhibiteurs, dans le réacteur, 1,09 g de TOPANOL A, et, dans la colonne, une solution de TOPANOL A à 5% et d'HQ à 5% dans l'acide acétique, cette solution étant ajoutée régulièrement pendant toute la durée de la réaction à raison de 2 ml/h.

Un bullage d'air appauvri (8% d'oxygène et 92% d'azote en volumes) est maintenu dans le réacteur durant toute la durée de l'opération.

L'acide acétique formé est éliminé au fur et à mesure de sa formation. La première fraction distillée est composée de 99,5% d'acide acétique.

Après 6 heures 30 de réaction à 95°C, le brut réactionnel a la composition suivante :

| | |
|---|---|
| AcOH | 0,5 % |
| Ac₂O | 0,06 % |
| AMA | 11,8 % |
| Mixte | 4,6 % |
| AMA₂O | 81,6 % |
| Produits secondaires | 1,44 % |
| TOTAL | 100 % |

La quantité d'AMA₂O contenue dans le brut (mesurée par pesée en fin de réaction) est de 485 g.

Ainsi, le taux de conversion de Ac₂O en AMA₂O et Mixte est de 96,5%.

Le rapport molaire final R_{f} AMA/Ac₂O est de 2,1 ;il est identique au rapport molaire initial.

Le brut obtenu est ensuite étêté sous pression réduite (20 mm Hg) afin d'en éliminer l'AMA résiduel et le Mixte.

Le brut obtenu après étêtage est constitué de 96,2% d'AMA₂O.

### Exemple 2 (selon l'invention)

On procède comme indiqué dans l'Exemple 1, sauf que l'on charge dans le réacteur la même masse (1000 g) de charge globale initiale d'AMA et d'Ac₂O, mais en introduisant plus d'AMA et moins d'Ac₂O.

La charge initiale est donc constituée de 900 g (10,465 moles) d'AMA et de 100 g (0,98 mole) d'Ac₂O (le rapport molaire initial R₀ est de 10,7).

On introduit dans le réacteur le même inhibiteur qu'à l'Exemple 1 et dans la même proportion.

On introduit dans la colonne la même solution d'inhibiteurs qu'à l'Exemple 1 et avec le même débit.

Tout au long de la réaction, on introduit régulièrement de l'Ac₂O, au fur et à mesure de l'élimination de l'AcOH, de manière à occuper toute la place libérée par celui-ci dans le réacteur.

Cette manière d'opérer permet une occupation optimale de la capacité réactionnelle.

Après 6 heures 30 de réaction à 95°C, la quantité d'Ac₂O introduite en continu au cours de la réaction a été de 408 g et le brut réactionnel a la composition suivante :

| | |
|---|---|
| AcOH | 0,4 % |
| Ac₂O | 0,2 % |
| AMA | 12,3 % |
| Mixte | 7,2 % |
| AMA₂O | 78,8 % |
| Produits secondaires | 1,44 % |
| TOTAL | 100 % |

La quantité d'AMA₂O contenue dans le brut (mesurée par pesée en fin de réaction) est de 680 g, pour une quantité totale de réactif mise en oeuvre de 1408 g (1000 + 408).

Le rapport molaire final R_{f} est de 2,1, comme dans l'Exemple 1.

Par conséquent, le gain de production d'AMA₂O est de 40% par rapport à l'Exemple 1 et cela, sans augmentation du temps de réaction et avec le même rapport molaire final R_{f}.

Ainsi, pour le même volume réactionnel (même masse initiale de 1000 g), mais avec une meilleure répartition des réactifs, on a produit beaucoup plus d'AMA₂O.

Le taux de conversion de Ac₂O en AMA₂O et Mixte est de 97%.

Le brut obtenu après étêtage est parfaitement limpide, exempt de polymères et apte à être distillé sous pression réduite (20 mm Hg). Il est constitué de 96,4% d'AMA₂O.

### Exemples 3 (comparatif) et 4 (selon l'invention)

On procède comme indiqué dans les Exemples 1 et 2, en remplaçant l'acide méthacrylique par l'acide acrylique et en utilisant une colonne à distiller ayant une efficacité de 20 plateaux théoriques.

Toutes les autres conditions sont identiques à celles des Exemples 1 et 2.

Au terme des 6 heures 30 de réaction à 95°C, on peut dresser le tableau suivant :

| Synthèse AA₂O | Exemple 3 (comparatif) | Exemple 4 (selon l'invention) |
|---|---|---|
| AA chargé au départ (g) | 535 | 735 |
| Ac₂O chargé au départ (g) | 361 | 161 |
| Total chargé au départ (g) | 896 | 896 |
| Ac₂O introduit en continu au cours de la réaction | 0 | 335 |
| Total chargé (g) pendant toute l'opération | 896 | 1231 |
| AA₂O produit (g) | 360 | 497 |

Le gain de production de l'Exemple 4 par rapport à l'Exemple 3 est donc de 38%.

## Revendications

1. Procédé de préparation d'anhydride (méth)acrylique en discontinu, dans lequel on fait réagir de l'anhydride acétique avec de l'acide (méth)acrylique et on élimine au moins en partie l'acide acétique au fur et à mesure de sa formation, **caractérisé en ce que** l'acide acétique éliminé est remplacé au moins partiellement par introduction dans le milieu réactionnel, au cours de la réaction, d'anhydride acétique et/ou d'acide (méth)acrylique;
à l'exclusion du procédé dans lequel le rapport molaire global de l'acide (méth)acrylique à l'anhydride acétique est compris entre 1 et 2 et dans lequel le rapport molaire initial Rₒ de l'acide (méth)acrylique à l'anhydride acétique est le double du rapport molaire global.

2. Procédé de préparation d'anhydride (méth)acrylique en discontinu, dans lequel on fait réagir de l'anhydride acétique avec de l'acide (méth)acrylique et on élimine au moins en partie l'acide acétique au fur et à mesure de sa formation, **caractérisé en ce que** l'acide acétique éliminé est remplacé au moins partiellement par introduction dans le milieu réactionnel, au cours de la réaction, d'anhydride acétique et/ou d'acide (méth)acrylique;
à l'exclusion du procédé mettant en oeuvre un catalyseur et un inhibiteur.

3. Procédé selon la revendication 1 ou 2, dans lequel le réactif introduit est l'anhydride acétique.

4. Procédé selon l'une des revendications 1 à 3, dans lequel le rapport molaire initial R₀ de l'acide (méth)acrylique à l'anhydride acétique est compris entre 2,5 et 11.

5. Procédé selon l'une des revendications 1 à 4, dans lequel le rapport molaire initial R₀ de l'acide (méth)acrylique à l'anhydride acétique est compris entre 9 et 11.

6. Procédé selon l'une des revendications 1 à 5, dans lequel le rapport molaire global de l'acide (méth)acrylique à l'anhydride acétique est compris entre 0,5 et 5.

7. Procédé selon l'une des revendications 1 à 6, dans lequel le rapport molaire global de l'acide (méth)acrylique à l'anhydride acétique est compris entre 1,8 et 2,2.

8. Procédé selon l'une des revendications 1 à 7, dans lequel tout l'acide acétique qui se forme pendant la réaction est éliminée au fur et à mesure de sa formation.

9. Procédé selon l'une des revendications 1 à 8, dans lequel la totalité de l'acide acétique éliminé est remplacée par l'anhydride (méth)acrylique et/ou d'acide (méth) acrylique.

10. Procédé selon l'une des revendications 1 à 9, dans lequel l'introduction d'anhydride acétique et/ou d'acide (méth)acrylique est réalisée de façon continue pendant toute la durée de la réaction.

11. Procédé selon l'une des revendications 1 à 10, dans lequel la réaction est mise en oeuvre dans un réacteur surmonté d'une colonne à distiller.

12. Procédé selon la revendication 11, dans lequel la colonne à distiller a une efficacité de séparation supérieure à 10 plateaux théoriques et de préférence supérieure à 12 plateaux théoriques.

13. Procédé selon l'une des revendications 1 à 12, dans lequel la réaction entre l'anhydride acétique et l'acide (méth)acrylique est mise en oeuvre en présence d'au moins un inhibiteur de polymérisation.

14. Procédé selon la revendication 13, lorsqu'elle est dépendante de la revendication 11 ou de la revendication 12, dans lequel on introduit au moins un inhibiteur dans le réacteur et au moins un inhibiteur dans la colonne à distiller.

15. Procédé selon la revendication 14, dans lequel :
- l'inhibiteur du réacteur est choisi dans le groupe constitué par le 2,4-diméthyl 6-terbutyl phénol, le 2,6-diterbutyl paracrésol et leurs mélanges ; et
- l'inhibiteur de la colonne à distiller est choisi dans le groupe constitué par l'hydroquinone, le le 2,4-diméthyl 6-terbutyl phénol, le 2,6-diterbutyl paracrésol, la phénotiazine et leurs mélanges.

16. Procédé selon la revendication 14 ou la revendication 15, dans lequel :
- l'inhibiteur du réacteur est introduit dans la charge initiale des réactifs, à raison d'au moins 0,001 % en poids ;
- l'inhibiteur de la colonne à distiller est introduit dans la colonne à distiller tout au long de la réaction.

17. Préparation de thioesters (méth)acryliques, d'amides (méth)acryliques, d'esters (méth)acryliques, **caractérisé en ce que** l'on met en oeuvre un anhydride (méth)acrylique et **en ce que** ledit anhydride (méth) acrylique est préparé selon l'une des revendications 1 à 16, par action de l'anhydride ascétique sur de l'acide (méth) acrylique, avec élimination au moins en partie de l'acide acétique au fur et à mesure de sa formation, et remplacement au moins partiellement par de l'anhydride acétique et/ou de l'acide (méth)acrylique.

## Patentansprüche

1. Verfahren zur diskontinuierlichen Herstellung von (meth)-acrylsäureanhydrid, bei dem man Essigsäureanhydrid mit (Meth)acrylsäure umsetzt und die Essigsäure wenigstens teilweise entsprechend ihrer Bildung entfernt, **dadurch gekennzeichnet, dass** die entfernte Essigsäure wenigstens teilweise durch zufuhr von Essigsäureanhydrid und/oder (Meth)-acrylsäure zum Reaktionsmedium im Laufe der Umsetzung ersetzt wird,
mit der Maßgabe, dass das Gesamtmolarverhältnis von (Meth)acrylsäure zu Essigsäureanhydrid nicht zwischen 1 und 2 liegt und das Ausgangsmolarverhältnis Rₒ der (Meth)acrylsäure zum Essigsäureanhydrid nicht das Doppelte des Gesamtmolarverhältnisses ausmacht.

2. Verfahren zur diskontinuierlichen Herstellung von (Meth)-acrylsäureanhydrid, bei dem man Essigsäureanhydrid mit (Meth)acrylsäure umsetzt und die Essigsäure wenigstens teilweise entsprechend ihrer Bildung entfernt, **dadurch gekennzeichnet, dass** die entfernte Essigsäure wenigstens teilweise durch Zufuhr von Essigsäureanhydrid und/oder (Meth)-acrylsäure zum Reaktionsmedium im Laufe der Umsetzung ersetzt wird,
mit der Maßgabe, dass kein Katalysator und kein Inhibitor verwendet werden.

3. Verfahren nach Anspruch 1 oder 2, bei dem das zugeführte Reagens Essigsäureanhydrid ist.

4. Verfahren nach einem der Ansprüche 1 bis 3, bei dem das Ausgangsmolarverhältnis Rₒ von (Meth)acrylsäure zu Essigsäureanhydrid zwischen 2,5 und 11 liegt.

5. Verfahren nach einem der Ansprüche 1 bis 4, bei dem das Ausgangsmolarverhältnis Rₒ von (Meth)acrylsäure zu Essigsäureanhydrid zwischen 9 und 11 liegt.

6. Verfahren nach einem der Ansprüche 1 bis 5, bei dem das Gesamtmolarverhältnis zwischen Methacrylsäure und Essigsäureanhydrid zwischen 0,5 und 5 liegt.

7. Verfahren nach einem der Ansprüche 1 bis 6, bei dem das Gesamtmolarverhältnis zwischen (Meth)acrylsäure und Essigsäureanhydrid zwischen 1,8 und 2,2 liegt.

8. Verfahren nach einem der Ansprüche 1 bis 7, bei dem die gesamte Essigsäure, die während der Umsetzung entsteht, entsprechend ihrer Bildung entfernt wird.

9. Verfahren nach einem der Ansprüche 1 bis 8, bei dem die gesamte entfernte Essigsäure durch (Meth)acrylsäureanhydrid und/oder (Meth)acrylsäure ersetzt wird.

10. Verfahren nach einem der Ansprüche 1 bis 9, bei dem die Zufuhr des Essigsäureanhydrids und/oder der (Meth)acrylsäure kontinuierlich während der gesamten Reaktionsdauer erfolgt.

11. Verfahren nnach einem der Ansprüche 1 bis 10, bei dem die Umsetzung in einem Reaktor, auf den eine Destillationskolonne aufgesetzt ist, durchgeführt wird.

12. Verfahren nach Anspruch 11, bei dem die Destillationskolonne eine Trennwirksamkeit von über 10 und vorzugsweise von über 12 theoretischen Böden aufweist.

13. Verfahren nach einem der Ansprüche 1 bis 12, bei dem die Umsetzung zwischen dem Essigsäureanhydrid und der (Meth)-acrylsäure in Anwesenheit wenigstens eines Polymerisationsinhibitors durchgeführt wird.

14. Verfahren nach Anspruch 13, wobei dieser von Anspruch 11 oder Anspruch 12 abhängt, bei dem man dem Reaktor wenigstens einen Inhibitor und der Destillationskolonne wenigstens einen Inhibitor zuführt.

15. Verfahren nach Anspruch 14, bei dem
- der Inhibitor des Reaktors ausgewählt wird aus der Gruppe, bestehend aus 2,4-Dimethyl-6-tert.-butyl-phenol, 2,6-Di-tert.-butyl-paracresol und ihren Gemischen, und
- der Inhibitor der Desillationskolonne ausgewählt wird aus der Gruppe, bestehend aus Hydrochinon, 2,4-Dimethyl-6-tert.-butylphenol, 2,6-Di-tert.-butyl-paracresol, Phenothiazin und ihren Gemischen.

16. Verfahren nach Anspruch 14 oder 15, bei dem
- der Inhibitor des Reaktors der Ausgangscharge der Reaktionsteilnehmer in einer Menge von wenigstens 0,001 Gew.-% zugeführt wird, und
- der Inhibitor der Destillationskolonne während der gesamten Reaktionsdauer der Destillationskolonne zugeführt wird.

17. Herstellung von (Meth)acrylthioestern, -amiden und -estern, **dadurch gekennzeichnet, dass** man ein (Meth)acrylsäureanhydrid verwendet und dieses nach einem der Ansprüche 1-16 durch Einwirkung von Essigsäureanhydrid auf (Meth)acrylsäure unter Entfernung wenigstens eines Teils der Essigsäure entsprechend ihrer Bildung und wenigstens teilweisen Ersatz durch Essigsäureanhydrid und/oder (Meth)acrylsäure herstellt.

## Claims

1. Process for the batchwise preparation of (meth)acrylic anhydride, in which acetic anhydride is reacted with (meth)acrylic acid and at least some of the acetic acid is removed gradually as it is formed, **characterized in that** the acetic acid removed is at least partly replaced by introducing into the reaction medium, during the reaction, acetic anhydride and/or (meth)acrylic acid;
with the proviso that the process is not one in which the overall molar ratio of the (meth)acrylic acid to the acetic anhydride is between 1 and 2 and in which the initial molar ratio R₀ of the (meth)acrylic acid to the acetic anhydride is twice the overall molar ratio.

2. Process for the batchwise preparation of (meth)acrylic anhydride, in which acetic anhydride is reacted with (meth) acrylic acid and at least some of the acetic acid is removed gradually as it is formed, **characterized in that** the acetic acid removed is at least partly replaced by introducing into the reaction medium, during the reaction, acetic anhydride and/or (meth)acrylic acid;
with the proviso that the process is not one in which a catalyst and an inhibitor are used.

3. Process according to Claim 1 or 2, in which the reagent introduced is acetic anhydride.

4. Process according to any one of Claims 1 to 3, in which the initial molar ratio R₀ of the (meth)acrylic acid to the acetic anhydride is between 2.5 and 11.

5. Procédé selon l'une des revendications 1 à 4, in which the initial molar ratio R₀ of the (meth)acrylic acid to the acetic anhydride is between 9 and 11.

6. Process according to any one of Claims 1 to 5, in which the overall molar ratio of the (meth)acrylic acid to the acetic anhydride is between 0.5 and 5.

7. Process according to any one of Claims 1 to 6, in which the overall molar ratio of the (meth)acrylic acid to the acetic anhydride is between 1.8 and 2.2.

8. Process according to any one of Claims 1 to 7, in which all the acetic acid which is formed during the reaction is removed gradually as it is formed.

9. Process according to any one of Claims 1 to 8, in which all the acetic acid removed is replaced with (meth)acrylic anhydride and/or (meth)acrylic acid.

10. Process according to any one of Claims 1 to 9, in which the introduction of acetic anhydride and/or of (meth)acrylic acid is performed continuously throughout the reaction.

11. Process according to any one of Claims 1 to 10, in which the reaction is carried out in a reactor on which is mounted a distillation column.

12. Process according to Claim 11, in which the distillation column has a separation efficacy of greater than 10 theoretical plates and preferably greater than 12 theoretical plates.

13. Process according to one of Claims 1 to 12, in which the reaction between the acetic anhydride and the (meth)acrylic acid is carried out in the presence of at least one polymerization inhibitor.

14. Process according to Claim 13, when it is dependent on Claim 11 or on Claim 12, in which at least one inhibitor is introduced into the reactor and at least one inhibitor is introduced into the distillation column.

15. Process according to Claim 14, in which:
- the reactor inhibitor is chosen from the group consisting of 2,4-dimethyl-6-tert-butylphenol and 2,6-di-tert-butyl-para-cresol, and the mixtures thereof; and
- the distillation column inhibitor is chosen from the group consisting of hydroquinone, 2,4-dimethyl-6-tert-butylphenol, 2,6-di-tert-butyl-para-cresol and phenothiazine, and mixtures thereof.

16. Process according to Claim 14 or Claim 15, in which:
- the reactor inhibitor is introduced into the initial charge of reagents, in a proportion of at least 0.001% by weight;
- the distillation column inhibitor is introduced into the distillation column throughout the reaction.

17. The preparation of (meth)acrylic thioesters, (meth)acrylic amides and (meth)acrylic esters **characterized in that** use is made of a (meth)acrylic anhydride and **in that** said (meth)acrylic anhydride is prepared by the process according to one of Claims 1 to 16 in which acetic anhydride is reacted with (meth)acrylic acid and the acetic acid is at least partly removed gradually as it is formed, and is at least partly replaced by acetic anhydride and/or (meth)acrylic acid.
